(19) Europäisches Patentamt
European Patent Office
Office européen des brevets

(11) **EP 3 292 813 B1**

(12) **EUROPEAN PATENT SPECIFICATION**

(45) Date of publication and mention
of the grant of the patent:
**25.06.2025 Bulletin 2025/26**

(51) International Patent Classification (IPC):
***A61B 5/024*** (2006.01)   ***A61B 5/00*** (2006.01)
***A61B 5/1455*** (2006.01)

(21) Application number: **17188409.1**

(22) Date of filing: **29.08.2017**

(52) Cooperative Patent Classification (CPC):
**A61B 5/02416; A61B 5/14551; A61B 5/721;**
**A61B 5/7257**

(54) **METHOD AND DEVICE FOR PROCESSING BIO-SIGNALS**

VERFAHREN UND VORRICHTUNG ZUR VERARBEITUNG VON BIOSIGNALEN

PROCÉDÉ ET DISPOSITIF DE TRAITEMENT DE SIGNAUX BIOLOGIQUES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.08.2016 PCT/IB2016/055158**

(43) Date of publication of application:
**14.03.2018 Bulletin 2018/11**

(73) Proprietor: **SmartCardia SA**
**1015 Lausanne (CH)**

(72) Inventors:
• **MURALI, Srinivasan**
  **1012 Lausanne (CH)**
• **RINCON VALLEJOS, Francisco Javier**
  **1020 Renens (CH)**

(74) Representative: **Linhart, Friedrich Karl Eberhard**
**La Couronnette**
**1166 Perroy (CH)**

(56) References cited:
**US-A1- 2015 100 141     US-A1- 2016 007 864**
**US-A1- 2016 022 220     US-A1- 2016 051 158**

EP 3 292 813 B1

**Description**

CROSS-REFERENCE TO RELATED APPLICATIONS

The present application claims foreign priority to International Patent Application PCT/IB2016/055158 filed on August 29, 2016.

TECHNICAL FIELD

The present invention relates to a method and a device for processing bio-signals to obtain heart rate and oxygen saturation under activity.

BACKGROUND

Accurate tracking of vital signs plays an important role in determining the health status of individuals. Typical hospital grade monitors measure one or more vital signs, such as the ElectroCardioGram (ECG or EKG), pulse signal, oxygen saturation and arterial blood pressure. To derive clinical insights from the different bio-signals, the important features need to be extracted. One of the major challenges in wearable and ambulatory bio-signal monitoring is to obtain accurate features under different noise sources, such as motion artefact due to activity, electromagnetic interference and power supply noise.

Photoplethysmography (PPG) based wrist worn or body worn sensors are widely used for heart rate monitoring. PPG sensing involves transmitting light waves at one or more wavelengths, and detecting the pulsatile variations due to blood flow in the reflectance of the signals.

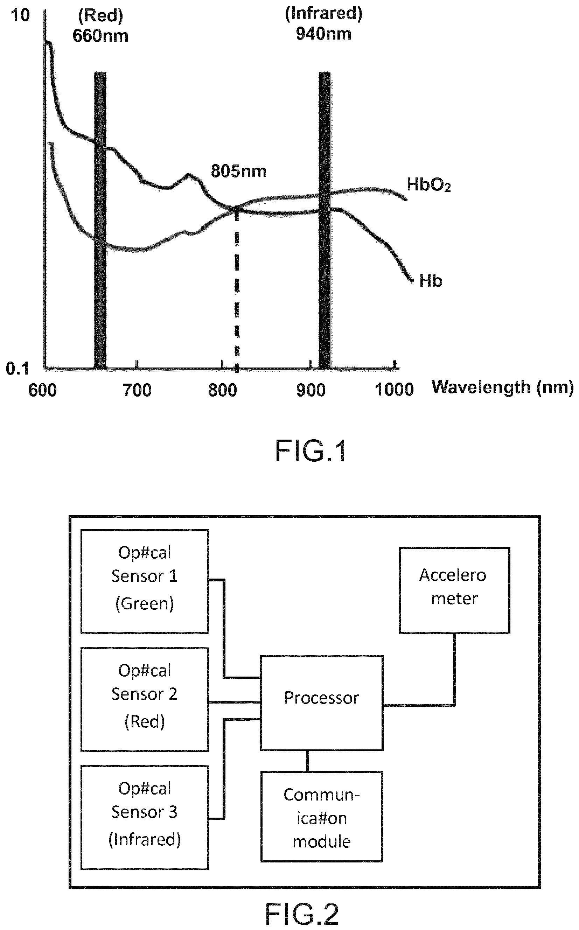

Arterial oxygen saturation (SaO2) level signifies the amount of oxygen-saturated hemoglobin relative to total hemoglobin in the blood. While SaO2 levels are typically obtained from testing the blood samples, the peripheral oxygen saturation values (SpO2) are a good approximation of SaO2 values. A SpO2 sensor, typically used at the fingertip, transmits light at two different wavelengths (Red and Infrared range) and measures the absorption patterns at the two wavelengths. In FIG. 1, the absorption of the light waves at different wavelengths due to oxygenated and non-oxygenated hemoglobin is shown in International Patent Publication WO 2013/038296.

The SpO2 value is calculated in a ratio-metric manner, by measuring the dissimilar absorption coefficients to oxygenated and deoxygenated hemoglobin. The R/IR values of SpO2 sensor is calculated using the following equation:

$$\frac{R}{IR} = \left( \frac{AC_R}{DC_R} \middle/ \frac{AC_{IR}}{DC_{IR}} \right) \qquad\qquad \text{(Equation 1)}$$

where $AC_R$ and $DC_R$ are the AC (pulsatile) and DC components of the sensed Red waveform, and $AC_{IR}$ and $DC_{IR}$ are the AC (pulsatile) and DC components of the sensed Infra-red waveforms. The R/IR values are, in some instances, calculated on a logarithmic scale (by taking a logarithm of numerator and denominator of the AC/DC values of Red and Infrared in the right hand side of the above equation and dividing the logarithm values to get the ratios).

By measuring the R/IR values of different subjects at different oxygen saturation levels, such as using an altitude chamber to change the oxygen saturation levels, a calibration model can be built to equate the R/IR to measured SaO2 values or to pre-calibrated SpO2 sensor.

Several works have shown the use of a linear model to relate the two, such as the following:

$$SpO2 = a - b * R/IR, \qquad\qquad \text{(Equation 2)}$$

where a and b are constants that are obtained from the calibration model.

While the calculation of SpO2 has been performed for decades, the main challenge is to obtain accurate SpO2 values under physical activity, where there is a lot of noise generated from the movement of the user, and to obtain under low perfusion levels, when the sensing is performed at the wrist, arm, chest or other points in the body. In US 2016/007864 A1, examples of SpO2 and heart rate calculations including spectral analyses are provided. Accordingly, novel and substantially improved methods and devices are desired for the calculating the peripheral oxygen saturation values SpO2, and other types of bio-signals that are related to blood oxygen levels.

**EP 3 292 813 B1**

SUMMARY

The invention is defined in the appended claims. In a first aspect, this disclosure provides a method for computing the heart rate value of an user, including (a) a first detecting of a time-dependent optical waveform including a pulse induced by the user's heartbeat, with an optical pulse sensor attached to the user, (b) a second detecting of a time-dependent accelerometer waveform by means of an inertial sensor arranged in proximity with the optical pulse sensor, (c) a first computing of frequency components of the time-dependent optical waveform my means of a mathematical transform, (d) a second computing of frequency components of the time-dependent accelerometer waveform by means of the mathematical transform, (e) a first removing of the computed frequency components of the time-dependent accelerometer waveform that are below a first pre-defined threshold of the maximum amplitude across the frequency components of the accelerometer, (f) a second removing of the computed frequency components of the time-dependent optical waveform that are matching to the computed frequency components of the time-dependent accelerometer waveform that remain after the first removing, (g) a third removing of the computed frequency components after the second removing that are below a second pre-defined threshold of the maximum amplitude across the remaining frequency components after the second removing, and (h) choosing one of the computed frequency components from the third removing as the heart rate.

In a preferred embodiment, the inertial sensor has more than one axis, and the computing of the heart rate further includes (a) measuring activity across more than one dimension, (b) a third computing of the frequency components of the different axes of the accelerometer signal of the second detecting by means of the same mathematical transform as in the first detecting, (c) a fourth removing of the computed frequency components from the third computing that are below pre-defined thresholds of the maximum amplitude across the frequency components of each axis of the accelerometer, (d) a fifth removing of the computed frequency components of the pulse signal computed in the first computing that are matching to those frequency components of each axis of the accelerometer signal that remain after the fourth removing, (e) a sixth removing of the frequency components of the fifth removing that are below another pre-defined threshold of the maximum amplitude across the remaining frequency components of the pulse signal of the fifth removing, and (f) choosing one of the frequency components from the sixth removing as the heart rate.

In a further preferred embodiment, the step of choosing one the computed frequency components from the third removing as the hearth rate, the frequency component with the highest amplitude is chosen as the heart rate.

In a further preferred embodiment, the pulse signal and accelerometer signal are divided into multiple time windows, with the steps of claim 1 executed for each window, and for a particular window, in the step of choosing one of the computed frequency components from the third removing as the heart rate, the frequency component that is closest to the one chosen as the heart rate in the previous time window is selected as the heart rate for the window.

In a further preferred embodiment, the pulse signal and accelerometer signals are divided into multiple time windows, with the steps of the computing of the heart rate further including (a) measuring activity across more than one dimension, (b) a third computing of the frequency components of the different axes of the accelerometer signal of the second detecting by means of the same mathematical transform as in the first detecting, (c) a fourth removing of the computed frequency components from the third computing that are below pre-defined thresholds of the maximum amplitude across the frequency components of each axis of the accelerometer, (d) a fifth removing of the computed frequency components of the pulse signal computed in the first computing that are matching to those frequency components of each axis of the accelerometer signal that remain after the fourth removing, (e) a sixth removing of the frequency components of the fifth removing that are below another pre-defined threshold of the maximum amplitude across the remaining frequency components of the pulse signal of the fifth removing, and (f) choosing one of the frequency components from the sixth removing as the heart rate executed for each window, and for a particular window, in the step of choosing one of the frequency components from the third removing as the heart rate, the frequency component that is closest to the one chosen as the heart rate in the previous time window is selected as the heart rate for the window.

In a second aspect, this disclosure provides a method for computing the Spo2 value of a user, including (a) a first detecting of a time-dependent optical waveform including a pulse induced by a heartbeat of the user, with an optical pulse sensor attached to the user that transmits light at a first wavelength, (b) a second detecting of a time-dependent optical waveform including a pulse induced by the user's heartbeat, with an optical pulse sensor attached to the user that transmits light at a second wavelength, (c) a first computing of the DC value of the signal of the first detected waveform (d) a second computing of the DC value of the signal of the second detected waveform, (e) a third computing the heart rate using Steps 1(a)-1(h) using the first detected waveform, (f) a fourth computing of the frequency components of the first detected waveform and choosing the amplitude of the frequency component that matches the heart rate computed in step (e) as the AC value of the first detected waveform, (g) a fifth computing of the frequency components of the second detected waveform and choosing the amplitude of the frequency component that matches the heart rate computed in the step of the third computing as the AC value of the second detected waveform, and (h) a sixth computing of the ratio of the ratio of the AC value of the fourth computing to the DC value of the first computing to ratio of the AC value of the fifth computing to the DC value of the second computing, which is then transformed by a polynomial to obtain Spo2 value.

In a preferred embodiment of the method of Spo2 computation, (a) apart from the pulse signals at two different wavelengths computed in the steps of the first detecting and the second detecting, the method further includes detecting a time-dependent optical waveform including a pulse induced by the user's heartbeat with an optical sensor configured to attach to the user that transmits light at a third wavelength, and (b) the heart rate computation of the step of the third computing is performed using the pulse signal at the third wavelength.

In a further preferred embodiment of the method of Spo2 computation, the SpO2 computation is performed in windows, with the heart rate computation of the step of the third computing performed using at least the following steps: (a) measuring activity across more than one dimension, (b) third computing of the frequency components of the different axes of the accelerometer signal of the second detecting by means of the same mathematical transform as in the first detecting, (c) a fourth removing of the computed frequency components from the third computing that are below pre-defined thresholds of the maximum amplitude across the frequency components of each axis of the accelerometer, (d) a fifth removing of the computed frequency components of the pulse signal computed in the first computing that are matching to those frequency components of each axis of the accelerometer signal that remain after the fourth removing, and (e) a sixth removing of the frequency components of the fifth removing that are below another pre-defined threshold of the maximum amplitude across the remaining frequency components of the pulse signal of the fifth removing.

In a third aspect, this disclosure provides a device for estimating the heart rate of a user under activity including: (a) means of recording a time-dependent optical waveform pulse induced by the user's heartbeat with an optical pulse sensor configured to attach to the user, (b) means of recording a time-dependent accelerometer waveform including using an inertial sensor configured to be in proximity with the optical pulse sensor, (c) a processor that acquires the optical waveform and accelerometer waveform, with (i) first means for computing the frequency components of the optical signal using a mathematical transform; (ii) second means for computing the frequency components of the accelerometer signal using the same mathematical transform as the first means for computing, (iii) first means for removing all frequency components computed by the second means for computing that are below a pre-defined threshold of the maximum amplitude across the frequency components of the accelerometer, (iv) second means for removing all the frequency components of the pulse signal computed by the first means for computing that are matching to those frequency components of the accelerometer signal that remain after removal by the first means for removing, (v) third means for removing all frequency components resulting from the second means for removing that are below another pre-defined threshold of the maximum amplitude across the remaining frequency components of the pulse signal resulting from the second means for removing, (vi) means for choosing one of the frequency components from the third means for removing as the heart rate.

In a preferred embodiment of the device, (a) the accelerometer has more than one axis, measuring activity across more than one dimension, (b) means for computing the frequency components of the different axes of the accelerometer signal from the second means for computing use the same mathematical transform as the first means for computing; and the device further includes (c) fourth means for removing all frequency components of each axis of the accelerometer in the means for computing that are below pre-defined thresholds of the maximum amplitude across the frequency components of each axis of the accelerometer, (d) fifth means for removing all the frequency components of the pulse signal computed in the first means for computing that are matching to those frequency components of each axis of the accelerometer signal that remain after removal by the fourth means for removing, and (e) sixth means for removing all the frequency components from the second means for removing that are below another predefined threshold of the maximum amplitude across the remaining frequency components of the pulse signal in the fifth means for removing.

In a further preferred embodiment of the device, in the second means for removing, the frequency component with the highest amplitude is chosen as the heart rate.

In a further preferred embodiment of the device, the processor segments the pulse signal and accelerometer signal into multiple time windows, with the first and second means for computing and the first, second and third means for removing executed for each window, and for a particular window, in the means for choosing one of the frequency components, the frequency component that is closest to the one chosen as the heart rate in the previous time window is selected as the heart rate for the window.

In a fourth aspect this disclosure provides a device for computing the Spo2 value of an user, including (a) means for detecting a time-dependent optical waveform including a pulse induced by the user's heartbeat with an optical sensor configured to attach to the user that transmits light at a first wavelength, (b) means for detecting a time-dependent optical waveform including a pulse induced by the user's heartbeat with an optical sensor configured to attach to the user that transmits light at a second wavelength, (c) means for computing the DC value of the signal of the first wavelength, (d) means for computing the DC value of the signal of the second wavelength, (e) means for computing the heart rate using a processor that acquires the optical waveform and accelerometer waveform, with (i) first means for computing the frequency components of the optical signal using a mathematical transform, (ii) second means for computing the frequency components of the accelerometer signal using the same mathematical transform as the first means for computing, (iii) first means for removing all frequency components computed by the second means for computing that are below a pre-defined threshold of the maximum amplitude across the frequency components of the accelerometer, (iv) second means for removing all the frequency components of the pulse signal computed by the first means for computing that are matching to those frequency components of the accelerometer signal that remain after removal by the first means for removing, (v) third means for removing all frequency components resulting from the second means for removing that are below another pre-defined threshold of the maximum amplitude across the remaining frequency components of the pulse signal resulting from the second means for removing, (vi) means for choosing one of the frequency components from the third means for removing as the heart rate. using one of the pulse signal at the first wavelength, (f) means for computing the frequency components of the signal at the first wavelength and choosing the amplitude of the frequency component that matches the heart rate computed with the means for computing the heart rate as the AC value at the first wavelength, (g) means for computing the frequency components of the signal at the second wavelength and choosing the amplitude of the frequency component that matches the heart rate computed with the means for computing the heart rate as the AC value at the second wavelength, (h) means for computing the ratio of AC/DC at first wavelength to AC/DC at second wavelength, which is then used for calibration to obtain the SpO2 values.

In a preferred embodiment of the device for computing the Spo2 value (a) apart from the pulse signals at two different wavelengths computed in the means for detecting a time-dependent optical waveform and the means for detecting a time-dependent optical waveform, the device further comprises means of detecting a time-dependent optical waveform including a pulse induced by the user's heartbeat with an optical sensor configured to attach to the user that transmits light at a third wavelength, and (b) means for computing the heart rate enabled to perform using the pulse signal at the third wavelength.

In a further preferred embodiment of method of Spo2 computation, the signals are divided into time segments of activity and inactivity, based on the accelerometer values, and the SpO2 computations are performed individually in the different segments.

BRIEF DESCRIPTION OF THE SEVERAL VIEWS OF THE DRAWINGS

The accompanying drawings, which are incorporated herein and constitute part of this specification, illustrate the presently preferred embodiments of the invention, and together with the general description given above and the detailed description given below, serve to explain features of the invention.

FIG. 1 shows oxygenated versus de-oxygenated blood light absorption of IR and Red from source as shown in reference [5];

FIG. 2 schematically illustrates a wearable sensor with 3 optical sensors, accelerometer, an on-board processor and a communication module;

FIG. 3 schematically illustrates a wearable sensor with 3 optical sensors, electrical sensor (ECG), accelerometer, an on-board processor and a communication module;

FIG. 4 shows different example locations for wearing the device according to the invention on the body;

FIGs. 5A and 5B show different perspective views of the wearable device according to an example embodiment of the invention, that can obtain multiple wavelength pulse signals and/or ECG with inbuilt accelerometer, and configured to be attached using an arm band/wrist band or a chest patch, the armband having metal sensors on the band to acquire the ECG signal;

FIG. 6 depicts an example of a window of pulse signal under no or very low movement of the user;

FIG. 7 depicts an example of an FFT of the window, wherein a maximum amplitude peak signifies the pulse rate of around 72 bpm;

FIG. 8 depicts an exemplary flowchart illustrating an algorithm for determining heart rate using a window of pulse and accelerometer signal, according to an example embodiment of the invention;

FIG. 9 depicts an example of the FFT of the pulse signal under noise;

FIG. 10 depicts an example of the FFT of the accelerometer signal under noise, wherein t1 is a pre-defined threshold, the value of t1*maximum peak being shown by the horizontal line in the plot-the value of t1 is set experimentally, based on user data collection, in this example, 6 peaks are above the threshold;

FIG. 11 depicts an example of the FFT of the pulse signal, after removing the peaks from FIG. 9 (the crossed ones are peaks removed), with the threshold t2*maximum amplitude of remaining peaks marked by the horizontal line; and

FIG. 12 depicts an exemplary flowchart illustrating an algorithm for determining heart rate using a window of pulse and accelerometer signal from a three-axis accelerometer.

Herein, identical reference numerals are used, where possible, to designate identical elements that are common to the figures. Also, the images in the drawings are simplified for illustration purposes and may not be depicted to scale.

DETAILED DESCRIPTION OF THE EMBODIMENTS

First and overview of the different aspects of the invention is provided. According to one aspect of the present invention, a method and a device is provided to accurately determine heart rate under activity and SpO2 under activity under noisy ambulatory conditions and/or under low perfusion levels and/or with ambient noise sources (such as different light conditions, electrical interference and power supply noise).

Several methods have been proposed to measure heart rate under activity. In Z. Zhang, Z. Pi, S. Member, and B. Liu, "TROIKA : A General Framework for Heart Rate Monitoring Using Wrist-Type Photoplethysmographic ( PPG ) Signals During Intensive Physical Exercise," IEEE Transactions on Biomedical Engineering, Vol. 62, No. 2, pp. 522-531, 2015, hereinafter reference [1], a framework was presented for obtaining heart rate from wrist based PPG sensors under activity. They use a periodogram based transformation, followed by signal decomposition and sparse signal reconstruction. The method has high complexity to be implemented using a low power microcontroller. Another method is based on periodic moving average filter as shown in H.-W. Lee, J.-W. Lee, W.-G. Jung, and G.-K. Lee, "The Periodic Moving Average Filter for Removing Motion Artifacts from PPG Signals," International Journal Of Control Automation And Systems, Vol. 5, No. 6, pp. 701-706, 2007, hereinafter in reference [2]. The filter segments the PPG signal into periods and performs resampling in each period. One key issue with the method is that it is difficult to remove in-band noise, when the heart rate and activity have similar periodicity. Adaptive filters for motion compensation have been proposed in T. Tamura, Y. Maeda, M. Sekine, and M. Yoshida, "Wearable Photoplethysmographic Sensors Past and Present," Electronics, Vol. 3, No. 2, pp. 282-302, 2014, hereinafter reference [3]. Such methods typically require a reference signal to minimize the error between the filter output and reference. In patent application U.S. Pat. Pub. No. 2015/0131879, herein cited as reference [4], Independent Component Analysis (ICA) is used to detect the heart rate under activity.

In International Publication WO 2014/163583, herein cited as reference [6], a method and apparatus to determine SpO2 is presented. In this document, the authors describe a method to bin R-values into different predetermined frequency bins. It does not provide any methods for accurately estimating the R-value under activity. In U.S. Pat. No., 8,954,135, herein cited as reference [7], devices for measuring SpO2 using Green and Infrared pulse sensors are presented.

In International Publication WO2013/038296, herein cited as reference [5], a threshold-based method is presented for heart rate detection under activity. In the patent, a predefined threshold is used and when the bio-signal quality is above the threshold, the heart rate is calculated. This is different from the method according to some aspects of the present invention, where we present a method and device with thresholds that are used in the frequency domain for selecting the frequency domain peaks of accelerometer and pulse signal. In U.S. Pat. No. 8,998,815, a wearable heart rate monitor is presented in which the frequency components of the accelerometer and pulse sensor are used to detect the heart rate. The method does not have predefined thresholds to remove noise.

According to one aspect of the present invention, a low complexity method for heart rate estimation under activity is provided. In one embodiment of the invention, one or more optical sensors and inertial sensor, for example single or multiple axis accelerometer, are located in close proximity. The unit may be worn at different body parts, such as the upper arm, wrist and chest. The optical sensor is used to detect a time-dependent optical waveform including a pulse induced by the user's heartbeat attached to the user. The accelerometer is used to detect a time-dependent activity waveform. During physical activity, such as walking, running or movement, the pulse signal measured by the optical sensor is corrupted by the movement artefact. The movement artefact also has a combination of different noise sources, such as the muscle noise and movement of the sensor under activity. By cancelling the movement, measured using the accelerometer from the pulse signal, the heart rate (or pulse rate) due to the user's heart beat can be obtained.

In a further embodiment of the invention, the optical sensor may emit a green light and the reflecting pulsatile variations are measured. In another embodiment of the invention, two optical sensors that emit red and infrared light are used. In yet another embodiment of the invention, three optical sensors that emit green, red and infrared lights are used. The three optical sensors are sampled alternatively, so that three time-dependent optical waveforms are measured simultaneously.

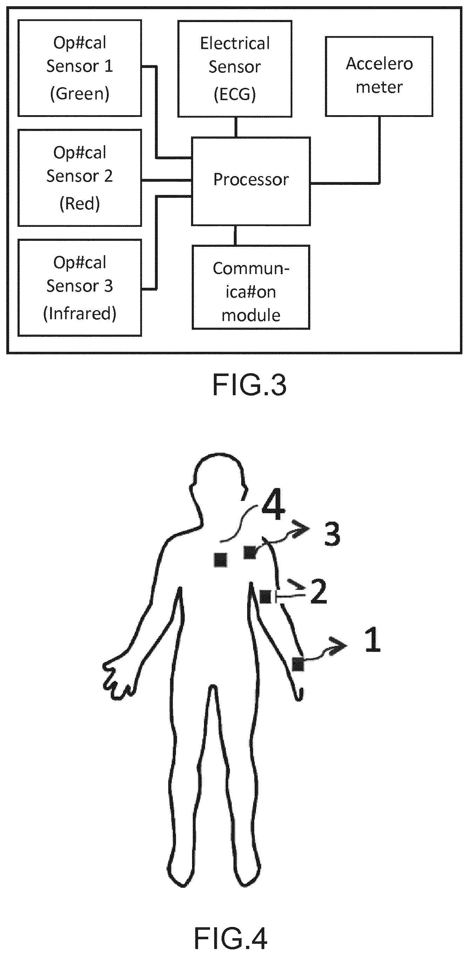

In another embodiment, an electrical sensor is used along with the optical sensors and the accelerometer. The electrical sensor uses two or more sensing pads and measures the ElectroCardioGram (ECG or EKG) of the user, at either the arm, wrist or chest. The wearable device architecture with the three optical sensors and accelerometer is shown in FIG. 2. The device also has an onboard processor to compute the heart rate, oxygen saturation values, and a communication module. The process can include but is not limited to a processor chip, microprocessor, a microcontroller, a computer processor, a programmable logic device, a field programmable gate array, and can also include memory that is operatively connected thereto. Standard protocols and communication interfaces, such as Bluetooth, Bluetooth Low Energy or wireless communication can be used to transit the data and signals from the device by the communication module. Moreover, it is also possible that computer instructions that are configured to perform the method are recorded on a non-transitory computer readable medium, for example but not limited to a CDROM, Bluray disc, DVD-ROM, memory stick, memory card, portable hard drive, hard drive, thumb drive, or other type of medium that can be operatively connected to a computer device, and can execute the method of computing the Spo2 value of a user, when the computer instructions are performed on the computer device. The wearable device architecture with the electrical sensor for measuring ECG as well is shown in FIG. 3.

The device may be worn in different parts of the body, as shown in FIG. 4. Some of the locations for wearing the device are the wrist (location 1), upper arm (location 2), on upper chest (location3) or sternum (location 4).

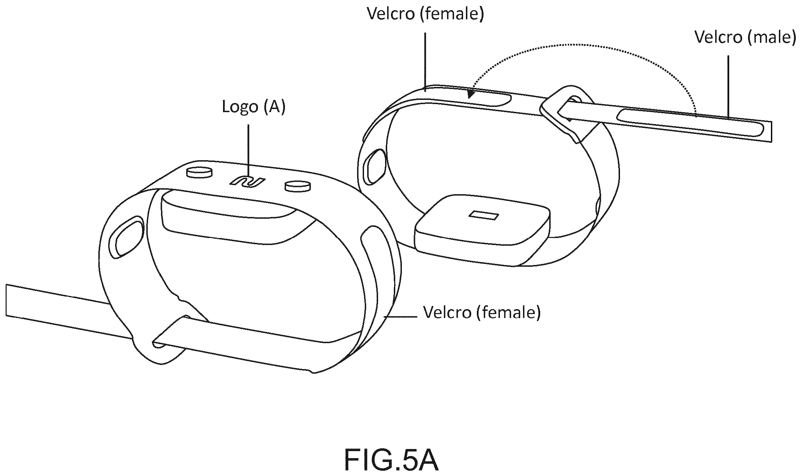

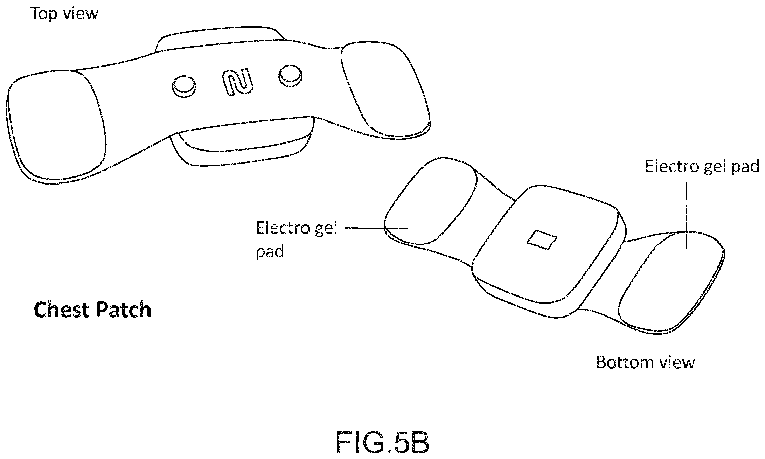

The snapshots of the exemplary device are shown in FIGs. 5A and 5B. More precisely, FIGs. 5A and 5B show perspective views of the wearable device that obtains multiple wavelength pulse signals and/or ECG with inbuilt accelerometer. The device may be attached using an arm band/wrist band or a chest patch. The armband has metal sensors on the band to acquire the ECG signal.

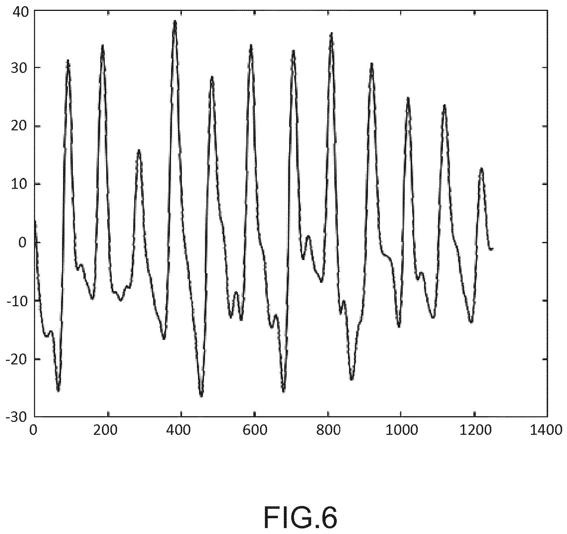

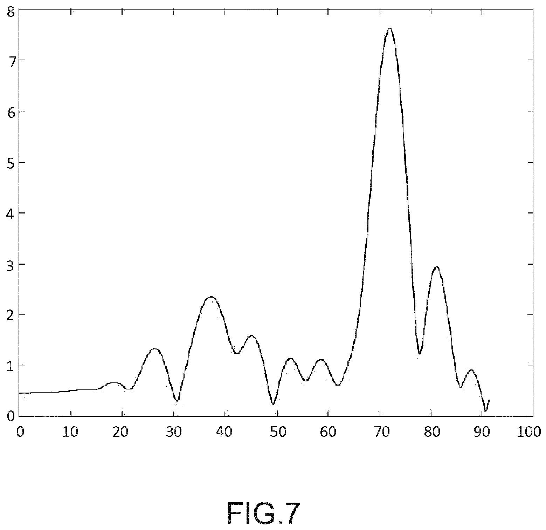

Next, the method for heart rate detection under activity is discussed, according to another aspect of the present invention. An example of the time varying pulse waveform is shown in FIG. 6, under no or very low movement of the user. One of the usual methods to determine the heart rate from the pulse signal is to perform the spectral analysis using Fast Fourier Transform (FFT). A FFT is performed on the signal, and when the pulse signal is not subject to noise sources, the peak of the FFT signifies the pulse rate (alternatively, also signified as heart rate). The FFT of the pulse signal from FIG. 6 is shown in FIG. 7. Each peak on the FFT denotes a frequency component of the signal and the amplitude of the peak (also termed as the amplitude of the frequency component) denotes the intensity of the periodicity of the signal at that frequency. When there is no motion artifact and other noise sources, the frequency component of the pulse signal corresponding to the heart rate value has the highest intensity or amplitude.

The X-axis of the FFT (in FIG. 6) is the rate, the Y-axis represents the amplitude of the signal at that rate. Each peak of the FFT also denotes a frequency component of the underlying signal. When there is no activity and no noise, the maximum amplitude peak (or the frequency component with the highest amplitude) signifies the heart rate (around 72 bpm in FIG. 7).

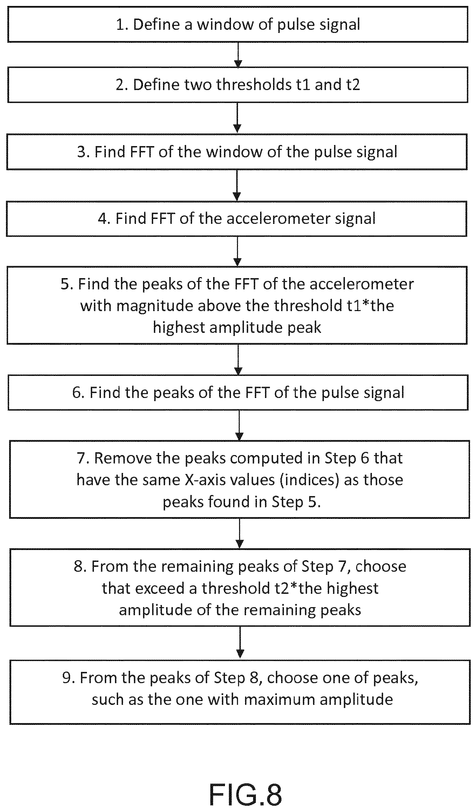

When the subject is under activity, the movement artefact also appears on the pulse signal. In the invention, the accelerometer data is used to remove the movement artefact from the pulse signal. In one embodiment of the patent, the steps to determine heart rate under activity are shown in a flowchart in FIG. 8.

The method involves taking the frequency transform of the pulse and accelerometer signal (such as the FFT), defining thresholds t1 and t2 for accelerometer and pulse signal, removing the peaks of the FFT of the accelerometer below the threshold t1, removing the remaining peaks from the FFT of the pulse signal, removing the remaining peaks of the FFT of the pule signal below the threshold t2 and choosing one of the remaining peaks of the FFT of the pulse signal.

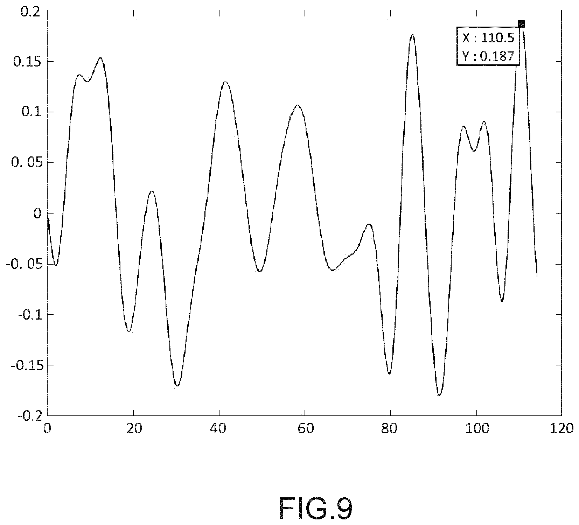

An example of the FFT of the pulse signal and accelerometer is shown in FIG. 9, and the method is explained using an example shown in FIGs. 9-12.

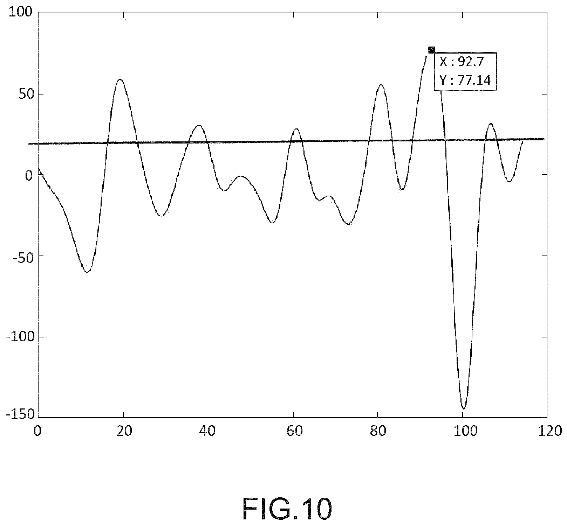

FIG. 10 shows an example of the FFT of the accelerometer signal under noise. t1 is a pre-defined threshold. The value of t1*maximum peak is shown by the horizontal line in the plot. The value of t1 is set experimentally, based on user data collection. In the above example, 6 peaks are above the threshold.

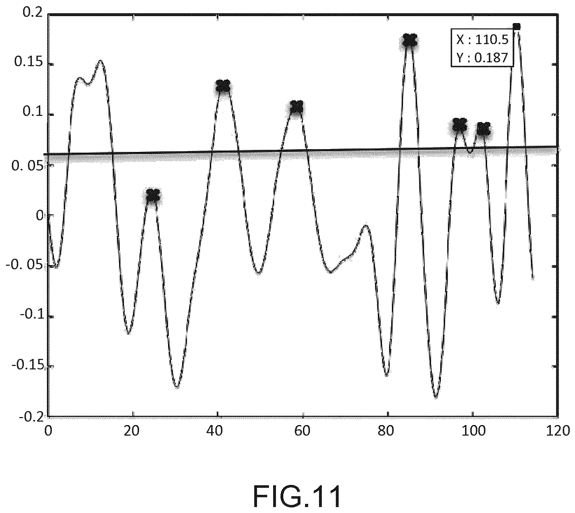

FIG. 11 shows an example of the FFT of the pulse signal, after removing the peaks from FIG. 9 (the crossed ones are peaks removed), with the threshold t2*maximum amplitude of remaining peaks marked by the horizontal line.

In a further embodiment of the invention, the thresholds t1 and t2 are based on training data, where different values in the range of [0,1] are used and the ones that lead to accurate heart rate (when compared to a gold standard measurement, for example using ECG measurement on the chest) measurements are used. In another embodiment, the accelerometer frequency components are removed from the pulse frequency components only when the raw value of the accelerometer exceeds a pre-defined threshold. This is to avoid the case of accidentally removing the real heart rate frequency component when the accelerometer does not experience any significant movement.

In the above algorithm, if multi-axis accelerometer is used (such as the 3D accelerometer), then in step 5, the peaks of the FFT of each accelerometer axis is detected and removed from the pulse FFT peaks (in step 7). The threshold t1 can be same for all the axes of the accelerometer, or individual thresholds can be set for each axes.

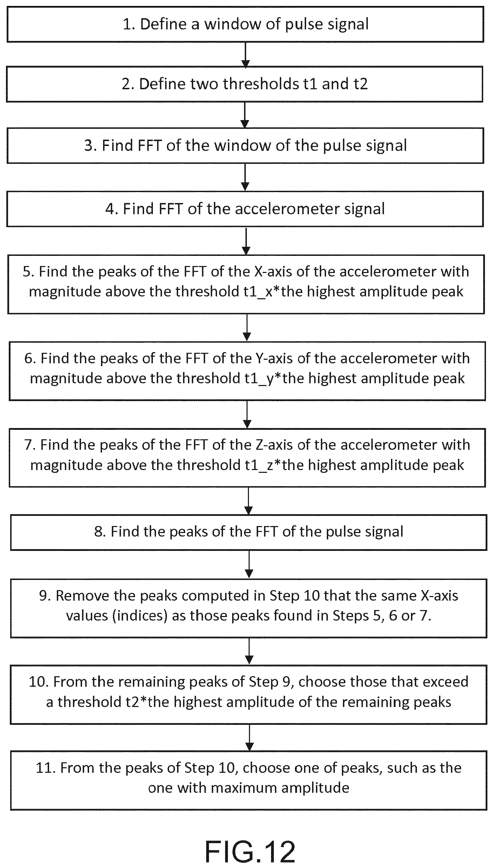

In FIG. 12, we show the application of the algorithm when using a 3-axis accelerometer that measures the activity across the X-axis, Y-axis and Z-axis. We use 3 thresholds, t1_x, t1_y and t1_z, apart from the threshold t2 for the pulse signal. In one embodiment of the patent, the three thresholds (t1_x, t1_y and t1_z) can be equal.

In another embodiment of the invention, the 3 different accelerometer values are combined to a single one, such as by taking a RMS (Root Mean Squared) value. The RMS signal is then used as the accelerometer signal in the algorithm of FIG. 8.

The above algorithms are defined for a single window of PPG and accelerometer signals. According to an aspect of the present invention, the heart rate continuously computed in real-time, either on the device or on an external data processing device that receives the data, for example a mobile phone, tablet or a smart phone. Moreover, the heart rate can be displayed on a device screen or monitor.

In this case, the above algorithm can be extended as follows: processing to accumulate signals in window: A moving window is used, where in signals samples from the PPG sensor and accelerometer are stored for particular time window. Then, the above algorithms are applied. The window is then time shifted, ignoring a set of older values and using newer values.

In a further embodiment of the invention, a window of eight (8) seconds is used. The moving window is shifted by 1-second and the algorithms are implemented. In this case, the oldest 1 second values are discarded and the newest 1-second are added. Thus, the heart rate is computed every second, using the last 8-seconds of data.

In a further embodiment of the invention, from the resulting peaks in Step 9 (of FIG. 8) or Step 11 (of FIG. 12), the peak that is closest to the peak chosen in the previous window is selected.

Next, the method for detecting oxygen saturation (SpO2) is explained with non-limiting embodiments. In order to detect the SpO2, the following steps can be performed on a processor of a computer:

1. Define a window of the Red and Infrared PPG signals
2. Compute the DC value of the Red and Infrared signals (RDC and IRDC)
3. Compute the AC value of Red and Infrared signals (RAC and IRAC)
4. Compute the ratio of R/IR with the following Equation 3:

$$\frac{R}{IR} = \left( \frac{\frac{AC_R}{DC_R}}{\frac{AC_{IR}}{DC_{IR}}} \right)$$

(Equation 3)

In a further embodiment of the invention, the DC value (in Step 1 above) is computed as the mean of the PPG signal. In another embodiment of the patent, the DC value is computed to be the amplitude of the FFT of the 0th component (at X-axis value of 0) of the PPG signal.

In another embodiment of the invention, the AC value (in Step 3 above) is computed as the maximum amplitude of the FFT of the signals.

Because the PPG signals can have significant baseline wander, in another embodiment of the invention, the AC values are computed as the maximum amplitude of the FFT of the signal after subtracting the mean of the signal (removing part of the baseline wander).

In another embodiment of the invention, the AC values are computed as the maximum amplitude of the FFT of the signal after filtering the signal (removing part of the baseline wander) by a band pass filter. In another embodiment, the particular bandpass filter used has a cut-off of [0.5Hz,4Hz].

In a further embodiment of the invention, the heart rate (Step 9 of FIG. 8, or Step 11 of FIG. 12) is computed using one of the Red or Infrared signals in a window, and the AC values (of Red and Infrared) are calculated as the amplitudes of the FFT of the signals (Red and Infrared, respectively) at that particular heart-rate. That is, the amplitude of the FFT with X-axis matching the heart rate.

In another embodiment of the invention, the heart rate, exemplarily shown in step 9 of FIG. 8, or step 11 of FIG. 12, is computed using one of the Red or Infrared signals in a window, and the AC values (of Red and Infrared) are calculated as the amplitudes of the FFT of the signals (Red and Infrared, respectively) after subtracting the mean, at that particular heart-rate. That is, the amplitude of the FFT with X-axis matching the heart rate.

In still another embodiment of the invention, the heart rate, for example step 9 of FIG. 8, or step 11 of FIG. 12, is computed using one of the Red or Infrared signals in a window, and the AC values (of Red and Infrared) are calculated as the amplitudes of the FFT of the signals (Red and Infrared, respectively) after filtering the signals, at that particular heart-rate. That is, the amplitude of the FFT with X-axis matching the heart rate.

In a further embodiment of the invention, we use a different signal to compute heart rate, and find the amplitude of the signal in the Red and Infrared sensor signals and compute SpO2. As green light PPG sensor has better skin penetration than Red and Infrared PPG sensors, this is preferred to obtain heart rate.

In another embodiment of the invention, the heart rate (Step 9 of FIG. 8, or Step 11 of FIG. 12) is computed using a Green LED PPG sensor in a window, and the AC values (of Red and Infrared) are calculated as the amplitudes of the FFT of the signals (Red and Infrared, respectively) after filtering the signals, at that particular heart-rate computed from the Green LED PPG sensor. That is, the amplitude of the FFT with X-axis matching the heart rate.

In a further embodiment of the invention, the device measures ECG, Green LED PPG, Red LED PPG and Infrared LED PPG at the same time.

In another embodiment of the invention, the heart rate is computed from an ECG signal in the window, and the AC values (of Red and Infrared) are calculated as the amplitudes of the FFT of the signals (Red and Infrared, respectively) after filtering the signals, at that particular heart rate computed from the ECG. That is, the amplitude of the FFT with X-axis matching the heart rate.

Next, a device for determining the heart rate is discussed, according to another aspect of the present invention. In another embodiment of the invention, the wearable device (of FIG. 2 or FIG. 3) is used for estimating the heart rate of a user under activity including (a) means of recording a time-dependent optical waveform pulse induced by the patient's heartbeat with an optical sensor configured to attach to the user, (b) means of recording a time-dependent accelerometer waveform including using an inertial sensor configured to be in proximity with the optical sensor of (a), and (c) a processor that acquires the optical waveform and accelerometer waveform, and performs the hearth rate computation according to the methods in FIG. 8 or FIG. 12. The means of recording the time dependent signals can include but are not limited to an analog-to-digital converter operatively coupled to the optical sensor or the inertial sensor or both, analog signal electronics for filtering, amplifying, and pre-processing the analog signals from the sensor, a communication line such as a cable or wireless communication between the sensor and the processor, memory buffer for temporarily storing the converted sensor data, an interface for providing the sensor data to the processor.

In another embodiment of the invention, the wearable device (of FIG. 2 or FIG. 3) is used for estimating the heart rate of a user under activity including (a) means of recording a time-dependent optical waveform pulse induced by the patient's heartbeat with an optical sensor configured to attach to the user, (b) means of recording a time-dependent accelerometer waveform including using an inertial sensor configured to be in proximity with the pulse sensor of (a), and (c) a processor that acquires the optical waveform and accelerometer waveform in windows, and performs the heart rate computation according to the methods in FIG. 8 or FIG. 12 for each window of the acquisition.

In another embodiment of the invention, the window can be sliding in nature, where a for each window, some old pulse and accelerometer values are discarded and newer ones are used, and the heart rate computation is performed.

Next a device for determining oxygen saturation is discussed, according to still another aspect of the invention.

In another embodiment of the invention, the wearable device (of FIG. 2 or FIG. 3) is used for estimating the oxygen saturation value of a user under activity including (a) means for detecting a time-dependent optical waveform including a pulse induced by the patient's heartbeat with an optical sensor configured to attach to the patient that transmits light at a first wavelength, (b) means for detecting a time-dependent optical waveform including a pulse induced by the patient's heartbeat with an optical sensor configured to attach to the patient that transits light at a second wavelength, (c) a processor that acquires the optical waveform and accelerometer waveform, and performs the methods to determine oxygen saturation on the processor, as shown above with respect to the method for detecting oxygen saturation. The means of detecting the time-dependent signals from the optical sensor can include but are not limited to an analog-to-digital converter operatively coupled to the optical sensor, analog signal electronics for filtering, amplifying, and pre-processing the analog signals from the sensor, a communication line such as a cable or wireless communication between the sensor and the processor for data transmission, memory buffer for temporarily storing the converted sensor data, an interface for providing the sensor data to the processor.

In another embodiment of the invention, the wearable device (of FIG. 2 or FIG. 3) is used for estimating the oxygen saturation value of a user under activity including (a) means for detecting a time-dependent optical waveform including a pulse induced by the patient's heartbeat with an optical sensor configured to attach to the patient that transmits light at a first wavelength, (b) means for detecting a time-dependent optical waveform including a pulse induced by the patient's heartbeat with an optical sensor configured to attach to the patient that transmits light at a second wavelength, (c) a processor that acquires the optical waveform and accelerometer waveform in windows, and performs the methods to determine oxygen saturation on the processor on each window, as shown above with respect to the method for detecting oxygen saturation.

In another embodiment of the invention, the window can be sliding in nature, where for each window, some old pulse and accelerometer values are discarded and newer ones are used, and the oxygen saturation computation is performed.

**Claims**

1. A method for computing the Spo2 value of a user, comprising:

   (a) a first detecting of a time-dependent optical waveform comprising a pulse induced by the user's heartbeat, with an optical pulse sensor attached to the user that transmits light at a first wavelength;
   (b) a second detecting of a time-dependent optical waveform comprising a pulse induced by the user's heartbeat, with an optical pulse sensor attached to the user that transmits light at a second wavelength;
   (c) a first computing of the DC value of the signal of the first detected waveform;
   (d) a second computing of the DC value of the signal of the second detected waveform;
   (e) a third computing the heart rate using the steps:

      (i) a first detecting of a third time-dependent optical waveform comprising a pulse induced by the user's heartbeat, with an optical pulse sensor attached to the user that transmits light at a third wavelength;

(ii) a second detecting of a time-dependent accelerometer waveform by means of an inertial sensor arranged in proximity with the optical pulse sensor that transmits light at a third wavelength;

(iii) a first computing of frequency components of the third time-dependent optical waveform by means of a mathematical transform;

(iv) a second computing of frequency components of the time-dependent accelerometer waveform by means of the mathematical transform;

(v) a first removing of the computed frequency components of the time-dependent accelerometer waveform that are below a first pre-defined threshold of the maximum amplitude across the frequency components of the accelerometer;

(vi) a second removing of the computed frequency components of the third time-dependent optical waveform that are matching to the computed frequency components of the time-dependent accelerometer waveform that remain after the first removing;

(vii) a third removing of the computed frequency components after the second removing that are below a second pre-defined threshold of the maximum amplitude across the remaining frequency components after the second removing; and

(viii) choosing one of the computed frequency components from the third removing as the heart rate;

(f) a fourth computing of the frequency components of the first detected waveform and choosing the amplitude of the frequency component that matches the heart rate computed in step of the third computing as the AC value of the first detected waveform;

(g) a fifth computing of the frequency components of the second detected waveform and choosing the amplitude of the frequency component that matches the heart rate computed in the step of the third computing as the AC value of the second detected waveform; and

(h) a sixth computing of the ratio of the ratio of the AC value of the fourth computing to the DC value of the first computing to ratio of the AC value of the fifth computing to the DC value of the second computing, which is then transformed by a polynomial to obtain Spo2 value.

2. The method of claim 1, wherein the inertial sensor has more than one axis, and wherein the computing of the heart rate further comprises

(a) measuring activity across more than one dimension;

(b) a third computing of the frequency components of the different axes of the accelerometer signal of the second detecting by means of the same mathematical transform as in the first detecting;

(c) a fourth removing of the computed frequency components from the third computing that are below pre-defined thresholds of the maximum amplitude across the frequency components of each axis of the accelerometer;

(d) a fifth removing of the computed frequency components of the pulse signal computed in the first computing that are matching to those frequency components of each axis of the accelerometer signal that remain after the fourth removing;

(e) a sixth removing of the frequency components of the fifth removing that are below another pre-defined threshold of the maximum amplitude across the remaining frequency components of the pulse signal of the fifth removing; and

(f) choosing one of the frequency components from the sixth removing as the heart rate.

3. The method of claim 1 wherein the step of choosing one the computed frequency components from the third removing as the hearth rate, the frequency component with the highest amplitude is chosen as the heart rate.

4. The method of claim 1 wherein the pulse signal and accelerometer signal are divided into multiple time windows, with the steps of claim 1 executed for each window, and for a particular window, in the step of choosing one of the computed frequency components from the third removing as the heart rate, the frequency component that is closest to the one chosen as the heart rate in the previous time window is selected as the heart rate for the window.

5. The method of claim 2 wherein the pulse signal and accelerometer signals are divided into multiple time windows, with the steps of claim 2 executed for each window, and for a particular window, in the step of choosing one of the frequency components from the third removing as the heart rate, the frequency component that is closest to the one chosen as the heart rate in the previous time window is selected as the heart rate for the window.

6. The method of any of the claims 1 to 5, wherein the SpO2 computation is performed in windows, with the heart rate computation of the step of the third computing performed using the following steps:

(a) measuring activity across more than one dimension;
(b) third computing of the frequency components of the different axes of the accelerometer signal of the second detecting by means of the same mathematical transform as in the first detecting;
(c) a fourth removing of the computed frequency components from the third computing that are below pre-defined thresholds of the maximum amplitude across the frequency components of each axis of the accelerometer;
(d) a fifth removing of the computed frequency components of the pulse signal computed in the first computing that are matching to those frequency components of each axis of the accelerometer signal that remain after the fourth removing; and
(e) a sixth removing of the frequency components of the fifth removing that are below another pre-defined threshold of the maximum amplitude across the remaining frequency components of the pulse signal of the fifth removing.

7. The method of claim 6, wherein the signals are divided into time segments of activity and inactivity, based on the accelerometer values, and the SpO2 computations are performed individually in the different segments.

8. A device for computing the Spo2 value of a user, comprising:

(a) means for detecting a time-dependent optical waveform comprising a pulse induced by the user's heartbeat with an optical sensor configured to attach to the user that transmits light at a first wavelength;
(b) means for detecting a time-dependent optical waveform comprising a pulse induced by the user's heartbeat with an optical sensor configured to attach to the user that transmits light at a second wavelength;
(c) means for computing the DC value of the signal of the first wavelength;
(d) means for computing the DC value of the signal of the second wavelength;
(e) means for computing the heart rate using a processor that acquires the optical waveform and accelerometer waveform, including,

(i) first means for computing the frequency components of the optical signal using a mathematical transform;
(ii) second means for computing the frequency components of the accelerometer signal using the same mathematical transform as the first means for computing;
(iii) first means for removing all frequency components computed by the second means for computing that are below a pre-defined threshold of the maximum amplitude across the frequency components of the accelerometer;
(iv) second means for removing all the frequency components of the pulse signal computed by the first means for computing that are matching to those frequency components of the accelerometer signal that remain after removal by the first means for removing;
(v) third means for removing all frequency components resulting from the second means for removing that are below another pre-defined threshold of the maximum amplitude across the remaining frequency components of the pulse signal resulting from the second means for removing; and
(vi) means for choosing one of the frequency components from the third means for removing as the heart rate;

(f) means for computing the frequency components of the signal at the first wavelength and choosing the amplitude of the frequency component that matches the heart rate computed with the means for computing the heart rate as the AC value at the first wavelength;
(g) means for computing the frequency components of the signal at the second wavelength and choosing the amplitude of the frequency component that matches the heart rate computed with the means for computing the heart rate as the AC value at the second wavelength; and
(h) means for computing the ratio of AC/DC at first wavelength to AC/DC at second wavelength, which is then used for calibration to obtain the SpO2 values, wherein
(a) apart from the pulse signals at two different wavelengths computed in the means for detecting a time-dependent optical waveform and the means for detecting a time-dependent optical waveform, the device further comprises means of detecting a time-dependent optical waveform comprising a pulse induced by the user's heartbeat with an optical sensor configured to attach to the user that transmits light at a third wavelength; and
(b) means for computing the heart rate enabled to perform using the pulse signal at the third wavelength.

9. A non-transitory computer readable medium having computer instructions recorded thereon, the computer instructions configured to perform a method according to any of the claims 1 to 7 when the computer instructions are performed on a computer device.

**Patentansprüche**

1. Ein Verfahren zum Berechnen des Spo2-Werts eines Benutzers, umfassend:

(a) ein erstes Erfassen einer zeitabhängigen optischen Wellenform umfassend einen vom Herzschlag des Benutzers induzierten Puls, mit einem an dem Benutzer befestigten optischen Pulssensor, welcher Licht mit einer ersten Wellenlänge aussendet;

(b) ein zweites Erfassen einer zeitabhängigen optischen Wellenform umfassend einen vom Herzschlag des Benutzers induzierten Puls, mit einem an dem Benutzer befestigten optischen Pulssensor, welcher Licht mit einer zweiten Wellenlänge aussendet;

(c) ein erstes Berechnen des DC-Werts des Signals der ersten erfassten Wellenlänge;

(d) ein zweites Berechnen des DC-Werts des Signals der zweiten erfassten Wellenlänge;

(e) ein drittes Berechnen der Herzfrequenz unter Nutzung der Schritte:

(i) ein erstes Erfassen einer dritten zeitabhängigen optischen Wellenform umfassend einen vom Herzschlag des Benutzers induzierten Puls, mit einem an dem Benutzer befestigten optischen Pulssensor, welcher Licht mit einer dritten Wellenlänge aussendet;

(ii) ein zweites Erfassen einer zeitabhängigen Beschleunigungssensor-Wellenform mit Hilfe eines Inertialsensors, angeordnet in der Nähe des optischen Pulssensors, welcher Licht mit einer dritten Wellenlänge aussendet;

(iii) ein erstes Berechnen von Frequenzkomponenten der dritten zeitabhängigen optischen Wellenform mit Hilfe einer mathematischen Transformation;

(iv) ein zweites Berechnen von Frequenzkomponenten der zeitabhängigen Beschleunigungssensor-Wellenform mit Hilfe einer mathematischen Transformation;

(v) ein erstes Entfernen der berechneten Frequenzkomponenten der zeitabhängigen Beschleunigungssensor-Wellenform, die unter einem ersten vordefinierten Schwellenwert der maximalen Amplitude von den Frequenzkomponenten des Beschleunigungssensors liegen;

(vi) ein zweites Entfernen der berechneten Frequenzkomponenten der dritten zeitabhängigen optischen Wellenform, die den berechneten Frequenzkomponenten der zeitabhängigen Beschleunigungssensor-Wellenform entsprechen, die nach dem ersten Entfernen übrig bleiben;

(vii) ein drittes Entfernen der berechneten Frequenzkomponenten nach dem zweiten Entfernen, die unter einem zweiten vordefinierten Schwellenwert der maximalen Amplitude von den restlichen Frequenzkomponenten nach dem zweiten Entfernen liegen; und

(viii) Auswählen einer der berechneten Frequenzkomponenten aus dem dritten Entfernen als die Herzfrequenz;

(f) ein viertes Berechnen der Frequenzkomponenten der ersten erfassten Wellenform und Auswahl der Amplitude der Frequenzkomponente, die der im Schritt des dritten Berechnens berechneten Herzfrequenz entspricht, als AC-Wert der ersten erfassten Wellenform;

(g) ein fünftes Berechnen der Frequenzkomponenten der zweiten erfassten Wellenform und Auswahl der Amplitude der Frequenzkomponente, die der im Schritt des dritten Berechnens berechneten Herzfrequenz entspricht, als AC-Wert der zweiten erfassten Wellenform; und

(h) ein sechstes Berechnen des Verhältnisses des Verhältnisses des AC-Wertes des vierten Berechnens zum DC-Wert des ersten Berechnens zum Verhältnis des AC-Wertes des fünften Berechnens zum DC-Wert des zweiten Berechnens, das dann durch ein Polynom transformiert wird, um Spo2-Wert zu erhalten.

2. Das Verfahren nach Anspruch 1, wobei der Inertialsensor mehr als eine Achse hat, und wobei das Berechnen der Herzfrequenz ferner umfasst

(a) Messung von Aktivität über mehr als einer Dimension;

(b) ein drittes Berechnen der Frequenzkomponenten der verschiedenen Achsen des Beschleunigungssensor-Signals des zweiten Erfassens mittels der gleichen mathematischen Transformation wie beim ersten Erfassen;

(c) ein viertes Entfernen der berechneten Frequenzkomponenten aus der dritten Berechnung, die unter vordefinierten Schwellenwerten der maximalen Amplitude von den Frequenzkomponenten jeder Achse des Beschleunigungssensors liegen;

(d) ein fünftes Entfernen der berechneten Frequenzkomponenten des beim ersten Berechnen berechneten Pulssignals, die jenen Frequenzkomponenten jeder Achse des Beschleunigungssensor-Signals entsprechen, die nach dem vierten Entfernen übrig bleiben;

(e) ein sechstes Entfernen der Frequenzkomponenten des fünften Entfernens, die unter einem anderen vordefinierten Schwellenwert der maximalen Amplitude von den übrigen Frequenzkomponenten des Pulssignals des fünften Entfernens liegen; und

(f) Auswählen einer der Frequenzkomponenten aus dem sechsten Entfernen als Herzfrequenz.

3. Das Verfahren nach Anspruch 1 wobei der Schritt des Auswählens einer der berechneten Frequenzkomponenten aus dem dritten Entfernen als Herzfrequenz, die Frequenzkomponente mit der höchsten Amplitude wird als Herzfrequenz gewählt.

4. Das Verfahren nach Anspruch 1 wobei das Pulssignal und Beschleunigungssensor-Signal in mehrere Zeitfenster unterteilt werden, wobei die Schritte des Anspruchs 1 für jedes Fenster ausgeführt werden, und für ein bestimmtes Fenster in dem Schritt des Auswählens einer der berechneten Frequenzkomponenten aus dem dritten Entfernen als die Herzfrequenz die Frequenzkomponente, die der im vorherigen Zeitfenster als Herzfrequenz ausgewählten am nächsten liegt, als die Herzfrequenz für das Fenster ausgewählt wird.

5. Das Verfahren nach Anspruch 2 wobei das Pulssignal und Beschleunigungssensor-Signal in mehrere Zeitfenster unterteilt werden, wobei die Schritte des Anspruchs 2 für jedes Fenster ausgeführt werden, und für ein bestimmtes Fenster in dem Schritt des Auswählens einer der berechneten Frequenzkomponenten aus dem dritten Entfernen als die Herzfrequenz die Frequenzkomponente, die der im vorherigen Zeitfenster als Herzfrequenz ausgewählten am nächsten liegt, als die Herzfrequenz für das Fenster ausgewählt wird.

6. Das Verfahren nach einem der Ansprüche 1 bis 5, wobei die SpO2-Berechnung in Fenstern ausgeführt wird, wobei das Herzfrequenz-Berechnen des Schritts des dritten Berechnens unter Benutzung der folgenden Schritte durchgeführt wird:

(a) Aktivitätsmessung über mehr als eine Dimension;

(b) ein drittes Berechnen der Frequenzkomponenten der unterschiedlichen Achsen des Beschleunigungssensor-Signals des zweiten Erfassens mittels der gleichen mathematischen Transformation wie beim ersten Erfassen;

(c) ein viertes Entfernen der berechneten Frequenzkomponenten aus der dritten Berechnung, die unter vordefinierten Schwellenwerten der maximalen Amplitude von den Frequenzkomponenten jeder Achse des Beschleunigungssensors liegen;

(d) ein fünftes Entfernen der berechneten Frequenzkomponenten des beim ersten Berechnen berechneten Pulssignals, die jenen Frequenzkomponenten jeder Achse des Beschleunigungssensor-Signals entsprechen, die nach dem vierten Entfernen übrig bleiben; und

(e) ein sechstes Entfernen der Frequenzkomponenten des fünften Entfernens, die unter einem anderen vordefinierten Schwellenwert der maximalen Amplitude von den übrigen Frequenzkomponenten des Pulssignals des fünften Entfernens liegen.

7. Das Verfahren nach Anspruch 6, wobei die Signale auf der Grundlage der Beschleunigungssensor-Werte in Zeitsegmente von Aktivität und Inaktivität unterteilt werden und die SpO2-Berechnungen individuell in den verschiedenen Segmenten durchgeführt werden.

8. Ein Gerät zum Berechnen des Spo2-Werts eines Benutzers, umfassend:

(a) Mittel zur Erfassung einer zeitabhängigen optischen Wellenform umfassend einen vom Herzschlag des Benutzers induzierten Puls mit einem optischen Sensor, konfiguriert zum Anbringen am Benutzer, der Licht mit einer ersten Wellenlänge aussendet;

(b) Mittel zur Erfassung einer zeitabhängigen optischen Wellenform umfassend einen vom Herzschlag des Benutzers induzierten Puls mit einem optischen Sensor, konfiguriert zum Anbringen am Benutzer, der Licht mit einer zweiten Wellenlänge aussendet;

(c) Mittel zum Berechnen des DC-Werts des Signals der ersten Wellenlänge;

(d) Mittel zum Berechnen des DC-Werts des Signals der zweiten Wellenlänge;

(e) Mittel zum Berechnen der Herzfrequenz mit Hilfe eines Prozessors, der die optische Wellenform und Beschleunigungssensors-Wellenform erfasst, umfassend,

(i) erste Mittel zum Berechnen der Frequenzkomponenten des optischen Signals unter Verwendung einer mathematischen Transformation;

(ii) zweite Mittel zum Berechnen der Frequenzkomponenten des Beschleunigungssensor-Signals unter Verwendung derselben mathematischen Transformation wie die ersten Mittel zur Berechnung;

(iii) erste Mittel zum Entfernen aller von den zweiten Mitteln zum Berechnen berechneten Frequenzkomponenten, die unter einem vordefinierten Schwellenwert der maximalen Amplitude von den Frequenzkomponenten des Beschleunigungsmessers liegen;

(iv) zweite Mittel zum Entfernen aller Frequenzkomponenten des durch das erste Mittel zum Berechnen berechneten Pulssignals, die den Frequenzkomponenten des Beschleunigungssensor-Signals entsprechen, die nach dem Entfernen durch die ersten Mittel zum Entfernen übrig bleiben;

(v) dritte Mittel zum Entfernen aller Frequenzkomponenten resultierend aus den zweiten Mitteln zum Entfernen, die unter einem weiteren vordefinierten Schwellenwert der maximalen Amplitude von den verbleibenden Frequenzkomponenten des Pulssignals liegen, das sich aus den zweiten Mitteln zum Entfernen ergibt; und

(vi) Mittel zur Auswahl einer der Frequenzkomponenten von den dritten Mitteln zum Entfernen als die Herzfrequenz;

(f) Mittel zum Berechnen der Frequenzkomponenten des Signals bei der ersten Wellenlänge und Wählen der Amplitude der Frequenzkomponente, die der Herzfrequenz entspricht, die mit dem Mittel zum Berechnen der Herzfrequenz berechnet wurde, als der AC-Wert bei der ersten Wellenlänge;

(g) Mittel zum Berechnen der Frequenzkomponenten des Signals bei der zweiten Wellenlänge und Wählen der Amplitude der Frequenzkomponente, die der Herzfrequenz entspricht, die mit den Mitteln zum Berechnen der Herzfrequenz berechnet wurde, als der AC-Wert bei der zweiten Wellenlänge; und

(h) Mittel zur Berechnung des Verhältnisses von AC/DC bei der ersten Wellenlänge zu AC/DC bei der zweiten Wellenlänge, das dann zur Kalibrierung verwendet wird, um die SpO2-Werte zu erhalten, wobei

(a) abgesehen von den Pulssignalen bei zwei verschiedenen Wellenlängen, die in den Mitteln zum Erfassen einer zeitabhängigen optischen Wellenform und den Mitteln zum Erfassen einer zeitabhängigen optischen Wellenform berechnet werden, umfasst das Gerät ferner Mittel zum Erfassen einer zeitabhängigen optischen Wellenform, die einen durch den Herzschlag des Benutzers induzierten Puls umfasst, mit einem optischen Sensor, konfiguriert zum Anbringen am Benutzer, der Licht mit einer dritten Wellenlänge aussendet; und

(b) Mittel zur Berechnung der Herzfrequenz, ausgebildet zum Ausführen unter Benutzung des Pulssignals bei der dritten Wellenlänge.

9. Ein nicht-transitorisches computerlesbares Medium mit darauf aufgezeichneten Computerbefehlen, wobei die Computerbefehle so konfiguriert sind, dass sie ein Verfahren nach einem der Ansprüche 1 bis 7 durchführen, wenn die Computerbefehle auf einem Computergerät ausgeführt werden.

**Revendications**

1. Un procédé de calcul de la valeur de SpO2 d'un utilisateur, comprenant :

(a) une première détection d'une forme d'onde optique dépendante du temps comprenant un pouls induit par le battement de cœur de l'utilisateur, avec un capteur optique de pouls attaché à l'utilisateur qui transmet de la lumière à une première longueur d'onde;

(b) une deuxième détection d'une forme d'onde optique dépendante du temps comprenant un pouls induit par le battement de cœur de l'utilisateur, avec un capteur optique de pouls attaché à l'utilisateur qui transmet de la lumière à une deuxième longueur d'onde;

(c) un premier calcul de la valeur DC du signal de la première forme d'onde détectée;

(d) un deuxième calcul de la valeur DC du signal de la deuxième forme d'onde détectée;

(e) un troisième calcul de la fréquence cardiaque utilisant les étapes:

(i) une première détection d'une troisième forme d'onde optique dépendante du temps comprenant un pouls induit par le battement de cœur de l'utilisateur, avec un capteur optique de pouls attaché à l'utilisateur qui transmet de la lumière à une troisième longueur d'onde;

(ii) une deuxième détection d'une forme d'onde d'accéléromètre dépendante du temps au moyen d'un capteur inertiel arrangé à proximité du capteur optique de pouls qui transmet de la lumière à une troisième longueur d'onde;

**14**

(iii) un premier calcul de composantes fréquentielles de la troisième forme d'onde optique dépendante du temps au moyen d'une transformation mathématique;

(iv) un deuxième calcul de composantes fréquentielles de la forme d'onde d'accéléromètre dépendante du temps au moyen de la transformation mathématique;

(v) une première élimination des composantes fréquentielles calculées de la forme d'onde d'accéléromètre dépendante du temps qui sont inférieures à un premier seuil prédéfini de l'amplitude maximale à travers les composantes fréquentielles de l'accéléromètre;

(vi) une deuxième élimination des composantes fréquentielles calculées de la troisième forme d'onde optique dépendante du temps qui correspondent aux composantes fréquentielles calculées de la forme d'onde de l'accéléromètre dépendante du temps restantes après la première élimination;

(vii) une troisième élimination des composantes fréquentielles calculées après la deuxième élimination qui sont inférieures à un deuxième seuil prédéfini de l'amplitude maximale à travers les composantes fréquentielles restantes après la deuxième élimination; et

(viii) choix de l'une des composantes fréquentielles calculées de la troisième élimination comme fréquence cardiaque;

(f) un quatrième calcul des composantes fréquentielles de la première forme d'onde détectée et choix de l'amplitude de la composante de fréquence correspondante à la fréquence cardiaque calculée à l'étape du troisième calcul comme la valeur AC de la première forme d'onde détectée;

(g) un cinquième calcul des composantes fréquentielles de la deuxième forme d'onde détectée et choix de l'amplitude de la composante de fréquence correspondante à la fréquence cardiaque calculée à l'étape du troisième calcul comme la valeur AC de la deuxième forme d'onde détectée; et

(h) un sixième calcul du rapport du rapport de la valeur AC du quatrième calcul à la valeur DC du premier calcul au rapport de la valeur AC du cinquième calcul à la valeur DC du deuxième calcul, qui est ensuite transformé par un polynôme pour obtenir la valeur Spo2.

2. Le procédé selon la revendication 1, où le capteur inertiel possède plus qu'un axe, et où le calcul de la fréquence cardiaque comprend en outre:

(a) mesurer de l'activité à travers plus qu'une dimension;

(b) un troisième calcul des composantes fréquentielles des différents axes du signal d'accéléromètre de la deuxième détection au moyen de la même transformation mathématique que lors de la première détection;

(c) une quatrième élimination des composantes fréquentielles calculées lors du troisième calcul qui sont inférieures à des seuils prédéfinis de l'amplitude maximale à travers les composantes fréquentielles de chaque axe de l'accéléromètre;

(d) une cinquième élimination des composantes fréquentielles calculées du signal de pouls calculé lors du premier calcul qui correspondent à celles composantes fréquentielles de chaque axe du signal d'accéléromètre qui restent après la quatrième élimination;

(e) une sixième élimination des composantes fréquentielles de la cinquième élimination qui sont inférieures à un autre seuil prédéfini de l'amplitude maximale à travers les composantes fréquentielles restantes du signal de pouls de la cinquième élimination; et

(f) choix de l'une des composantes fréquentielles de la sixième élimination comme fréquence cardiaque.

3. Le procédé selon la revendication 1, où l'étape de choix de l'une des composantes fréquentielles calculées de la troisième élimination comme fréquence cardiaque, la composante de fréquence avec l'amplitude la plus élevée est choisie comme fréquence cardiaque.

4. Le procédé selon la revendication 1, où le signal de pouls et signal d'accéléromètre sont divisés en plusieurs fenêtres temporelles, les étapes de la revendication 1 étant exécutées pour chaque fenêtre, et pour une fenêtre particulière, lors de l'étape de choix de l'une des composantes fréquentielles calculées de la troisième élimination comme la fréquence cardiaque, la composante de fréquence la plus proche de celle choisie comme fréquence cardiaque dans la fenêtre temporelle précédente est sélectionnée comme fréquence cardiaque pour la fenêtre.

5. Le procédé selon la revendication 2, où le signal de pouls et les signaux d'accéléromètre sont divisés en plusieurs fenêtres temporelles, les étapes de la revendication 2 étant exécutées pour chaque fenêtre, et pour une fenêtre particulière, dans l'étape de choix d'une des composantes fréquentielles de la troisième élimination comme la fréquence cardiaque, la composante de fréquence qui est la plus proche de celle choisie comme fréquence cardiaque dans la fenêtre temporelle précédente est sélectionnée comme la fréquence cardiaque pour la fenêtre.

**6.** Le procédé selon l'une quelconque des revendications 1 à 5, où le calcul de SpO2 est effectué dans des fenêtres, le calcul de la fréquence cardiaque de l'étape du troisième calcul étant effectué selon les étapes suivantes :

(a) mesurer de l'activité à travers plus qu'une dimension;
(b) troisième calcul des composantes fréquentielles des différents axes du signal d'accéléromètre de la deuxième détection au moyen de la même transformation mathématique que lors de la première détection;
(c) une quatrième élimination des composantes fréquentielles calculées du troisième calcul qui sont inférieures à des seuils prédéfinis de l'amplitude maximale à travers les composantes fréquentielles de chaque axe de l'accéléromètre;
(d) une cinquième élimination des composantes fréquentielles calculées du signal de pouls calculées lors du premier calcul qui correspondent à celles composantes fréquentielles de chaque axe du signal d'accéléromètre qui restent après la quatrième élimination; et
(e) une sixième élimination des composantes fréquentielles de la cinquième élimination qui sont inférieures à un autre seuil prédéfini de l'amplitude maximale à travers les composantes fréquentielles restantes du signal de pouls de la cinquième élimination.

**7.** Le procédé selon la revendication 6, où les signaux sont divisés en segments temporels d'activité et d'inactivité, sur la base des valeurs d'accéléromètre, et les calculs de SpO2 sont effectués individuellement dans les différents segments.

**8.** Un dispositif pour calculer la valeur de SpO2 d'un utilisateur, comprenant :

(a) des moyens pour détecter une forme d'onde optique dépendante du temps comprenant un pouls induit par le battement de cœur de l'utilisateur avec un capteur optique configuré pour s'attacher à l'utilisateur qui transmet de la lumière à une première longueur d'onde;
(b) des moyens pour détecter une forme d'onde optique dépendante du temps comprenant un pouls induit par battement de cœur de l'utilisateur avec un capteur optique configuré pour s'attacher à l'utilisateur qui transmet de la lumière à une deuxième longueur d'onde;
(c) des moyens pour calculer la valeur DC du signal de la première longueur d'onde;
(d) des moyens pour calculer la valeur DC du signal de la deuxième longueur d'onde;
(e) des moyens pour calculer la fréquence cardiaque en utilisant un processeur qui acquiert la forme d'onde optique et forme d'onde de l'accéléromètre, comprenant:

(i) des premiers moyens pour calculer les composantes fréquentielles du signal optique en utilisant une transformation mathématique;
(ii) des deuxièmes moyens pour calculer les composantes fréquentielles du signal de l'accéléromètre en utilisant la même transformation mathématique que les premiers moyens pour calculer;
(iii) des premiers moyens pour éliminer toutes composantes fréquentielles calculées par les deuxièmes moyens pour calculer qui sont inférieures à un seuil prédéfini de l'amplitude maximale à travers les composantes fréquentielles de l'accéléromètre;
(iv) des deuxièmes moyens pour éliminer toutes les composantes fréquentielles du signal de pouls calculées par les premiers moyens pour calculer qui correspondent à celles composantes fréquentielles du signal de l'accéléromètre qui restent après élimination par les premiers moyens pour éliminer;
(v) des troisièmes moyens pour éliminer toutes les composantes fréquentielles résultantes des deuxièmes moyens pour éliminer qui sont inférieures à un autre seuil prédéfini de l'amplitude maximale à travers les composantes fréquentielles restantes du signal de pouls résultant des deuxièmes moyens pour éliminer; et
(vi) des moyens pour choisir l'une des composantes fréquentielles des troisièmes moyens pour éliminer comme fréquence cardiaque;

(f) des moyens pour calculer les composantes fréquentielles du signal à la première longueur d'onde et choisir l'amplitude de la composante de fréquence qui correspond à la fréquence cardiaque calculée avec les moyens pour calculer la fréquence cardiaque comme la valeur AC à la première longueur d'onde ;
(g) des moyens pour calculer les composantes fréquentielles du signal à la deuxième longueur d'onde et choisir l'amplitude de la composante de fréquence qui correspond à la fréquence cardiaque calculée avec les moyens pour calculer la fréquence cardiaque comme la valeur AC à la deuxième longueur d'onde; et
(h) des moyens pour calculer le rapport AC/DC à la première longueur d'onde sur AC/DC à la deuxième longueur d'onde, qui est ensuite utilisé pour l'étalonnage afin d'obtenir les valeurs de SpO2, où
(a) en dehors des signaux de pouls à deux longueurs d'onde différentes calculés dans les moyens pour détecter une forme d'onde optique dépendante du temps et les moyens pour détecter une forme d'onde optique dépendante du temps, le dispositif comprend en outre des moyens de détection d'une forme d'onde optique dépendante du temps comprenant un pouls induit par le battement de cœur de l'utilisateur avec un capteur optique configuré pour s'attacher à l'utilisateur qui transmet de la lumière à une troisième longueur d'onde; et (b) des moyens pour calculer la fréquence cardiaque permettant d'effectuer en utilisant le signal de pouls à la troisième longueur d'onde.

9. Un support lisible par ordinateur non-transitoire sur lequel sont enregistrées des instructions informatiques, les instructions informatiques étant configurées pour exécuter un procédé selon l'une quelconque des revendications 1 à 7 lorsque les instructions informatiques sont exécutées sur un dispositif informatique.

**FIG.1**

**FIG.2**

FIG.3

FIG.4

FIG.5A

Top view

Electro gel pad

Electro gel pad

Bottom view

Chest Patch

FIG.5B

FIG.6

FIG.7

| 1. Define a window of pulse signal |
|---|

| 2. Define two thresholds t1 and t2 |
|---|

| 3. Find FFT of the window of the pulse signal |
|---|

| 4. Find FFT of the accelerometer signal |
|---|

| 5. Find the peaks of the FFT of the accelerometer with magnitude above the threshold t1*the highest amplitude peak |
|---|

| 6. Find the peaks of the FFT of the pulse signal |
|---|

| 7. Remove the peaks computed in Step 6 that have the same X-axis values (indices) as those peaks found in Step 5. |
|---|

| 8. From the remaining peaks of Step 7, choose that exceed a threshold t2*the highest amplitude of the remaining peaks |
|---|

| 9. From the peaks of Step 8, choose one of peaks, such as the one with maximum amplitude |
|---|

# FIG.8

FIG.9

FIG.10

FIG.11

1. Define a window of pulse signal

↓

2. Define two thresholds t1 and t2

↓

3. Find FFT of the window of the pulse signal

↓

4. Find FFT of the accelerometer signal

↓

5. Find the peaks of the FFT of the X-axis of the accelerometer with magnitude above the threshold t1_x*the highest amplitude peak

↓

6. Find the peaks of the FFT of the Y-axis of the accelerometer with magnitude above the threshold t1_y*the highest amplitude peak

↓

7. Find the peaks of the FFT of the Z-axis of the accelerometer with magnitude above the threshold t1_z*the highest amplitude peak

↓

8. Find the peaks of the FFT of the pulse signal

↓

9. Remove the peaks computed in Step 10 that the same X-axis values (indices) as those peaks found in Steps 5, 6 or 7.

↓

10. From the remaining peaks of Step 9, choose those that exceed a threshold t2*the highest amplitude of the remaining peaks

↓

11. From the peaks of Step 10, choose one of peaks, such as the one with maximum amplitude

## FIG.12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016055158 A
- WO 2013038296 A
- US 2016007864 A1
- US 20150131879 A
- WO 2014163583 A
- US 8954135 B
- US 8998815 B

**Non-patent literature cited in the description**

- **Z. ZHANG** ; **Z. PI** ; **S. MEMBER** ; **B. LIU**. TROIKA : A General Framework for Heart Rate Monitoring Using Wrist-Type Photoplethysmographic ( PPG ) Signals During Intensive Physical Exercise. *IEEE Transactions on Biomedical Engineering*, 2015, vol. 62 (2), 522-531
- **H.-W. LEE** ; **J.-W. LEE** ; **W.-G. JUNG** ; **G.-K. LEE**. The Periodic Moving Average Filter for Removing Motion Artifacts from PPG Signals. *International Journal Of Control Automation And Systems*, 2007, vol. 5 (6), 701-706
- **T. TAMURA** ; **Y. MAEDA** ; **M. SEKINE** ; **M. YOSHIDA**. Wearable Photoplethysmographic Sensors Past and Present. *Electronics*, 2014, vol. 3 (2), 282-302